# EUROPÄISCHE PATENTANMELDUNG

(11) **EP 4 687 212 A1**
(43) Veröffentlichungstag der Anmeldung: **04.02.2026**
(21) Anmeldenummer: 25166764.8
(22) Anmeldetag: 27.03.2025
(51) Int. Cl.: H01P 1/06, H01P 3/123

(54) **DATENÜBERTRAGUNGSSYSTEM FÜR COMPUTERTOMOGRAPHEN MIT EINEM WELLENLEITER**

(30) Priorität: 02.08.2024 DE 102024002510
(71) Anmelder: Venturetec Rotating Systems GmbH, 87600 Kaufbeuren (DE)
(72) Erfinder: Bubnov, Grigorii, 87600 Kaufbeuren (DE); Roßi, Philipp, 86860 Jengen (DE); Schilling, Harry, 91126 Schabach (DE); Neubauer, Stephan, 91301 Forchheim (DE)
(74) Vertreter: Henkel & Partner mbB

(57) **Zusammenfassung**

Eine erfindungsgemäße Vorrichtung zur Übertragung von Daten zwischen dem rotierenden Teil und dem stationären Teil eines Computertomographen mit einem in der Längsachse geschlitzten Hohlleiter zur Übertragung hochbitratiger Datensignale

## Beschreibung

Die Erfindung betrifft ein Datenübertragungssystem zur Übertragung von Daten zwischen zwei relativ zueinander um eine gemeinsame Achse drehbaren Teilen, beispielsweise einem rotierenden Teil und einem stationären Teil eines Drehübertragers, wie etwa bei einem Computertomographen, mittels eines geteilten Wellenleiters bzw. Hohlleiters.

Aus der US 6,433,631 ist eine Vorrichtung zur Datenübertragung in Computertomographen bekannt. Eine Streifenleitung im rotierenden Teil wird mit einem Sendersignal beaufschlagt. An einem stationären Teil ist ein Abgriff vorgesehen, welcher in einem geringen Abstand in einer Größenordnung von ca. 1 mm von der Streifenleitung geführt wird.

Die aus dem Stand der Technik bekannten Übertragungssysteme sind in den übertragbaren Datenraten auf Datenraten von max. 10 GBit/s begrenzt.

Weiterer Stand der Technik ist aus DE 35 38 035 A1 und DE 32 09 906 A1 bekannt.

Andere Vorrichtungen auf Basis traditioneller Wellenleitersysteme, die zur Übertragung von Hochfrequenzsignalen verwendet werden, weisen erhebliche Einschränkungen auf, insbesondere wenn sie in rotierenden Systemen eingesetzt werden. Bisherige Lösungen wie geschlitzte Wellenleiter bieten eine begrenzte Bandbreite und weisen eine hohe Einfügedämpfung auf, was die Übertragungsqualität beeinträchtigt.

Aufgabe der Erfindung ist es, ein Datenübertragungssystem vorzustellen, welches Datenübertragungsraten bis zu mehreren 100 GBit/s erlaubt und mit geringem mechanischem Aufwand zum Beispiel in Computertomographen integrierbar ist.

Eine erfindungsgemäße Lösung dieser Aufgabe ist in den unabhängigen Patentansprüchen angegeben. Weiterbildungen der Erfindung sind Gegenstand der abhängigen Ansprüche.

Die Erfindung wird nachstehend anhand von Ausführungsbeispielen unter Bezugnahme auf die Zeichnungen exemplarisch beschrieben.
- Fig. 1: zeigt schematisch eine Hohlleiteranordnung mit einem Stator und einem Rotor mit einem Steg gemäß einer ersten Ausführungsform der Erfindung.
- Fig. 2: zeigt schematisch eine Hohlleiteranordnung mit einem Stator und einem Rotor mit einem Steg gemäß einer zweiten Ausführungsform der Erfindung.
- Fig. 3: zeigt schematisch eine Hohlleiteranordnung mit einem Stator und einem Rotor mit mehreren Stegen gemäß einer dritten Ausführungsform der Erfindung.
- Fig. 4: zeigt schematisch eine Hohlleiteranordnung mit einem Stator und einem Rotor mit einem Absorber zur Terminierung des RF-Signals.
- Fig. 5: zeigt schematisch eine Hohlleiteranordnung mit einem Stator und einem Rotor mit einem Steg und einem knochenförmigen Hohlleiterquerschnitt gemäß einer weiteren Ausführungsform der Erfindung.
- Fig. 6: zeigt schematisch eine Hohlleiteranordnung mit einem Stator und einem Rotor mit einem Steg und einem kreisförmigen Hohlleiterquerschnitt gemäß einer weiteren Ausführungsform der Erfindung.
- Fig. 7: zeigt schematisch eine Hohlleiteranordnung mit einem Stator und einem Rotor mit mehreren Stegen und einem kreisförmigen Hohlleiterquerschnitt gemäß einer weiteren Ausführungsform der Erfindung.
- Fig. 8: zeigt schematisch eine Hohlleiteranordnung mit einem Stator und einem Rotor mit einem Steg und einem trapezförmigen, abgerundeten Hohlleiterquerschnitt gemäß einer weiteren Ausführungsform der Erfindung.
- Fig. 9: zeigt schematisch eine Hohlleiteranordnung mit einem Stator und einem Rotor mit mehreren Stegen und einem trapezförmigen, abgerundeten Hohlleiterquerschnitt gemäß einer weiteren Ausführungsform der Erfindung.

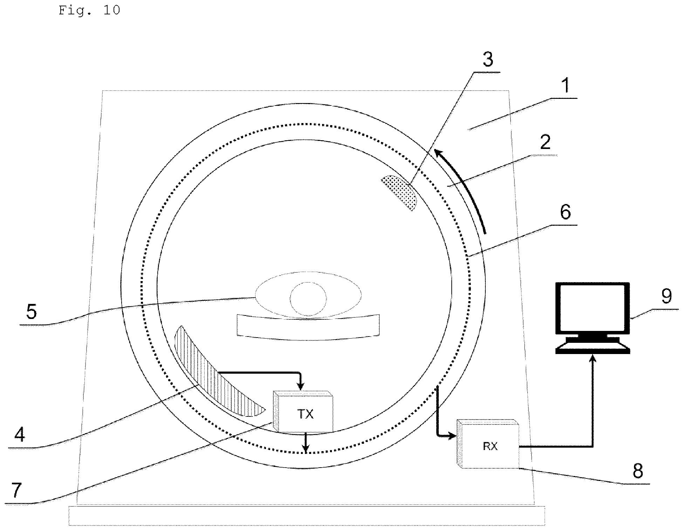

- Fig. 10: zeigt schematisch eine Anwendung des Hohlleiterübertragungssystems in einem Computertomographen.
- Fig. 11: zeigt ein detailliertes Beispiel zur Ausgestaltung der Terminierung eines erfindungsgemäßen Hohlleiters.
Im Folgenden wird die Erfindung detailliert am Beispiel eines Computertomographen beschrieben, ohne aber hierauf beschränkt zu sein.

Eine erfindungsgemäße Vorrichtung zur Übertragung von Daten zwischen einem rotierenden Teil oder Rotor 102 und einem stationären Teil oder Stator 103 eines Computertomographen umfasst beispielsweise eine Datenquelle am rotierenden Teil und wenigstens eine Datensenke am stationären Teil. Eine Datenquelle kann beispielsweise ein Röntgendetektor bzw. das Data-Acquisition-System (Datenverarbeitungssystem) oder eine Steuereinrichtung oder ein Rechner sein. Eine Datensenke kann ein Rechner zur Auswertung und Aufbereitung der Daten, aber auch eine andere Steuereinheit oder ein FPGA etc. sein.

Die Erfindung ist nicht auf die Anwendung bei einem Computertomographen beschränkt. Es ist auch nicht erforderlich, dass der Stator 103 tatsächlich und immer stationär ist. Es ist ausreichend, dass sich die beiden rotierenden Teile relativ zueinander um eine gemeinsame Achse drehen können. Auch ist die Übertragung der Daten nicht auf die Richtung vom Rotor 102 zum Stator 103 beschränkt. Auch die Übertragung in die umgekehrte Richtung und auch eine bidirektionale Übertragung sind möglich.

Weiterhin ist im Rotor 102 wenigstens eine Sendeeinrichtung oder ein Transmitter 7 sowie ein von dieser gespeister erster Wellenleiter 101, hier in der Form eines ringförmig um die gemeinsame Achse verlaufenden Hohlleiters, mit spezifischer Struktur und optimierten Abmessungen eingesetzt. Dieser Hohlleiter 101 ermöglicht eine verlustarme Übertragung von Hochfrequenzsignalen und reduziert die Dispersionseffekte, welche durch die Rotation verursacht werden.

Ein Hohlleiter ist ein leitfähiges, meist metallisches Rohr, das hochfrequente elektromagnetische Wellen leitet, typischerweise im Frequenzbereich von 1 bis 1500 GHz. Er umfasst einen Hohlraum, der durch leitende Wände nach außen abgegrenzt ist und keine Innenleiter enthält. Elektromagnetische Wellen breiten sich im Inneren des Hohlleiters aus, indem sie an den metallischen Wänden reflektiert werden. Die Wellen breiten sich in Moden innerhalb des Hohlleiters aus, wobei das elektrische Feld (E-Feld) und das magnetische Feld (H-Feld) senkrecht zueinander stehen und orthogonal zur Längsachse des Hohlleiters schwingen. Im Hohlleiter bildet sich ein elektrisches Feld in der Mitte der breiteren Seite (a) aus, das zu den schmaleren Seiten (b) hin abnimmt. Das magnetische Feld entsteht durch das elektrische Feld und kann nicht senkrecht auf der metallischen Wand stehen. Die Felder ändern ihre Intensität und Polarität im Rhythmus des Eingangssignals. Die Wellenausbreitung im Hohlleiter ist frequenzabhängig. Es gibt eine sogenannte Grenzfrequenz, unterhalb der keine Wellenausbreitung stattfindet. Diese Frequenz hängt von den Abmessungen des Hohlleiters ab, insbesondere von der Breite (a). Die Wellenlänge der zu übertragenden Welle muss kleiner als die Grenzwellenlänge sein, damit eine Ausbreitung möglich ist. Im Hohlleiter können sich verschiedene Moden (Wellentypen) ausbreiten, die als Hmn- bzw. Emn-Wellen bezeichnet werden. Diese Moden entstehen aus der Lösung der Maxwellschen Gleichungen unter den Randbedingungen des Hohlleiters. Die Grundwelle in einem rechteckigen Hohlleiter ist die H10-Welle, die bei der Wahl von b/a ≈ 0,5 über einen großen Frequenzbereich stabil ist. Hohlleiter werden in der Höchstfrequenz- und Mikrowellentechnik eingesetzt, da sie im Vergleich zu koaxialen Kabeln geringere Verluste aufweisen und große Leistungen übertragen können, ohne nennenswerte Verluste oder Spannungsdurchbrüche zu verursachen. Zusammenfassend ermöglicht ein Hohlleiter die nahezu verlustlose Fortleitung kurzer elektromagnetischer Wellen, wobei die Ausbreitung durch die Geometrie des Hohlleiters und die verwendeten Moden bestimmt wird.

Der Hohlleiter 101 ist so gestaltet, dass er die mechanische Bewegung zwischen Rotor 102 und Stator 103 ermöglicht, während die elektromagnetische Leistung effizient übertragen wird. Der Transmitter 7 empfängt Daten von der Datenquelle, etwa der Röntgenröhre 3, und setzt diese zur Leitung bzw. Kopplung in den Hohlleiter 101 in ein entsprechendes elektromagnetisches Signal um. Weiterhin ist im Stator 103 wenigstens eine Empfangseinrichtung oder ein Receiver 8 vorgesehen, welche das Signal aus dem Hohlleiter 101 empfängt bzw. auskoppelt. Der Receiver 8 setzt die Signale zur Weiterleitung an die Datensenke, z. B. einen Computer 9, um.

Die Signalübertragung vom Rotor 102 zum Stator 103 erfolgt mittels Signalleitung im Hohlleiter 101.

Die Signale können von dem Transmitter 7 moduliert und/oder kodiert werden.

Um eine Mehrwegeausbreitung zu vermeiden, kann durch eine modenselektive Einspeisung des Signals sichergestellt werden, dass das übertragene Signal klar und konsistent bleibt. Diese Technik minimiert auch die Auswirkungen des Dopplereffekts durch die Rotation des Drehübertragers und verbessert die Signalqualität.

Eine modenselektive Einspeisung wird durch optimierte T-Wellenleiterverbindungen erreicht, wobei die Abmessungen für ein besseres Stehwellenverhältnis (VSWR) optimiert sind.

Zur Minimierung der Kanalimpulsantwort und Vermeidung von Mehrwegeausbreitung wird bevorzugt eine Einzelmoden-Welle verwendet. Eine Mehrmoden-Ausbreitung würde aufgrund unterschiedlicher Ausbreitungsgeschwindigkeiten zu einer breiten Impulsantwort führen.

In der Erfindung ist der Hohlleiter 101 entlang der Längsachse geschlitzt bzw. geteilt. Beide, durch das Schlitzen entstehenden Teile des Hohlleiters 101 sind mit einem Spalt 6, 106 mit geringem Abstand zueinander, um die zentrale Drehachse beweglich angeordnet. Dies wird als Wellenleitersystem oder als Hohlleitersystem bezeichnet.

Der Hohlleiter 101 kann, basierend auf den notwendigen Übertragungseigenschaften, einen runden, schmetterlingsförmigen, T-förmigen, doppel-T-förmigen oder eckigen Querschnitt aufweisen, wie das in den verschiedenen Ausführungsformen der Erfindung in den Figuren gezeigt ist, wobei der eckige Querschnitt bevorzugt ist. Die Profile können dabei auch von der Grundform abweichen, indem Verrundungen oder Fasen angebracht werden.

Um Leckverluste an den Schlitzen des Hohlleiters 101 zu minimieren, werden im Querschnitt des Hohlleiters 101 einzelne oder mehrere Stege 105 eingebracht, welche die elektromagnetischen Felder konzentrieren und die Einfügedämpfung reduzieren. Die Stegstruktur optimiert die Modenausbreitung und minimiert die Verluste durch die fokussierte Konzentration des elektromagnetischen Feldes in der Mitte des Hohlleiters.

Die Stege 105 können etwa als einzelner Steg, doppelter Steg oder vierfacher Steg ausgeführt werden. Die Form und Dimensionen der Stege können dabei optimiert werden, um die Einfügedämpfung zu reduzieren und unerwünschte Moden zu unterdrücken.

Eine Ausgestaltung der Erfindung sieht im Stator 103 wenigstens einen Transmitter 7 und im Rotor 102 wenigstens einen Receiver 8 vor. Durch diese Ausgestaltung ist auch eine Kommunikation vom Stator 103 zum Rotor 102 möglich.

Eine weitere Ausgestaltung der Erfindung sieht mehrere Transmitter 7 und mehrere Receiver 8 vor, welche entweder jeweils auf einen von mehreren parallel angeordneten Hohlleitern 101 oder auf einen in mehrere Kreissegmente unterteilten Hohlleiter 101 angeordnet sind. Im letzteren Fall ist nicht notwendigerweise aber in bevorzugter Weise die Anzahl der Receiver 8 gleich n+1 und die Anzahl der Transmitter 8 gleich n.

Für bestimmte Anwendungen kann eine Terminierung 112 des Hohlleiters 101 erforderlich sein. Dies hängt von der Konfiguration und Anzahl der Transmitter 7 und Receiver 8 ab. Die Terminierung 112 erfolgt durch pyramidenförmiges Mikrowellenabsorbermaterial, das im Hohlleiter 101 positioniert ist.

Eine weitere Ausgestaltung der Erfindung sieht einen Hohlleiter 101 vor, welcher sowohl ein oder mehrere Signale vom Rotor 102 zum Stator 103 als auch vom Stator 103 zum Rotor 102 überträgt. Dabei können die Signale unterschiedliche Trägerfrequenzen verwenden und/oder unterschiedlich moduliert und/oder unterschiedlich kodiert sein.

Die Modulation kann beispielsweise eine Amplituden- oder Frequenzmodulation oder eine Mischung aus beiden wie z.B. QAM sein.

Für den Fall der gleichzeitigen Übertragung mehrerer Signale in einem Hohlleiter 101 ist sowohl im Transmitter 7 als auch im Receiver 8 eine Signal-/Frequenzweiche oder Ähnliches vorzusehen, welche durch geeignete Maßnahmen wie Frequenzselektion, Phasenselektion oder Richtungsselektion für eine Entkopplung zwischen den Signalen und Übertragungsrichtungen sorgt.

In einer weiteren Ausgestaltung der Erfindung wird das Sendesignal aufgrund eines oder mehrerer Auswahlparameter angepasst. Die Auswahlparameter können beispielsweise die Signalstärke, die Signalqualität wie zum Beispiel Rauschen, Amplitude, Error Vector Magnitude usw. sein.

In einer anderen Ausgestaltung ist die Erfindung als Array ausgeführt. Ein solches Array umfasst mehrere Hohlleiter 101, welche mit Signalen gespeist werden, die in einer definierten Relation zueinander stehen, um in der Gesamtheit ein bestimmtes Strahlungsdiagramm zu erhalten. Ein solches Array kann mit festeingestellten Phasenbeziehungen zwischen den einzelnen Strahlern oder auch mit variablen Phasenverhältnissen ausgeführt werden.

In einer Ausgestaltung ist eine Steuereinheit vorgesehen, welche, wenn gegeben, einzelne Hohlleitersegmente und die dazugehörigen Transmitter 7 und Receiver 8 nach vorgegebenen Parametern einstellt bzw. auswählt. Die vorgegebenen Parameter zur Einstellung bzw. Auswahl oder Selektion sind beispielsweise Signalpegel, Signal/Rauschabstand, Bitfehlerrate, Laufzeit und/oder Phasenverschiebung bezogen auf ein Referenzsignal oder auch ein Positionssignal.

Die Hohlleiter 101 werden aus leitfähigem Material, wie z.B. Metall, oder aus einem mit leitfähigem Material beschichteten Trägermaterial, z.B. Kunststoff, aufgebaut.

Im Falle der Verwendung von beschichteten Trägermaterial, kann es sinnvoll sein, dein Hohlleiter seitlich zu schlitzen, damit das Beschichtungsmaterial sich im Hohlleiter verteilen und an das Trägermaterial anlagern kann.

Vorzugsweise erfolgt die Beschichtung im Hohlleiter galvanisch oder auch chemisch.

Eine weitere Ausgestaltung der Erfindung sieht vor, dass zur Steuerung einer auf Zeitfenstern beruhenden bidirektionalen Kommunikation eine zusätzliche Steuereinheit vorgesehen ist, welche den Zeitrahmen für jede Kommunikationsrichtung vorgibt.

Eine weitere Ausgestaltung der Erfindung sieht vor, dass mindestens eine Schaltung zur Frame- und/oder Datenwiederherstellung vorgesehen ist.

Eine weitere Ausgestaltung der Erfindung umfasst einen Signalprozessor bzw. FPGA im Transmitter 7, welcher die Daten auf mehrere Hohlleiter 101 oder Hohlleitersegmente aufteilt, und eine elektronische Schaltung, z.B. ebenfalls beruhend auf einem Signalprozessor oder FPGA, in der Empfangseinheit, welche die Daten wieder zu einem Datenstrom zusammenführt.

Eine weitere Ausgestaltung der Erfindung umfasst einen Verstärker direkt an der Signaleinkopplung (Feed-In-Point) und/oder an der Signalauskopplung (Receiving Point) vor dem Receiver 8, wobei die Verstärkung des Verstärkers variabel ist und die Verstärkung anhand gemessener oder vorgegebener Parameter angepasst wird.

Eine weitere Ausgestaltung der Erfindung umfasst eine diskrete oder integrierte Auswerteschaltung, welche auf Basis verschiedener Qualitätskriterien, wie der Fehlerrate, bei mehreren Empfangseinheiten diejenige Empfangseinheit zur Weiterleitung des Signals auswählt, welche die Qualitätskriterien am besten erfüllt.

Zur Vereinfachung der Darstellung wird in diesem Dokument auf eine Übertragung von dem Rotor 102 auf den Stator 103 eines Computertomographen Bezug genommen. Selbstverständlich ist eine erfindungsgemäße Vorrichtung auch in umgekehrter Übertragungsrichtung einsetzbar. Ebenso kann eine erfindungsgemäße Vorrichtung auch in anderen Anwendungen zur Drehübertragung und ebenso zur linearen Übertragung zweier gegeneinander beweglicher Einheiten herangezogen werden.

Die Übertragungsrichtung entsprechend der Erfindung wurde vom Rotor 102 zum Stator 103 gewählt, da dies dem häufigsten Einsatzfall entspricht. Allerdings ist ebenso eine Übertragung in entgegengesetzter Richtung oder aber auch bidirektional möglich.

Die Erfindung stellt ein neuartiges Prinzip zur Übertragung von breitbandigen Mikrowellensignalen zwischen Rotor 102 und Stator 103 eines Drehübertragers vor. Ein wesentliches Merkmal dieses Ansatzes ist die Fähigkeit, einzelne breitbandige Wellen zu übertragen, die im Vergleich zu Multimode-Wellen kleinere Kanal-Impulsantworten aufweisen. Diese Besonderheit vereinfacht und erweitert die Möglichkeiten der Datenübertragung erheblich.

Das Datenübertragungssystem der Erfindung ist so konzipiert, dass es den insbesondere mit künftigen Photon-Counting-CT-Scanner-Anwendungen zugeordneten Anforderungen gerecht wird.

Hierzu gehört eine angestrebte hohe Datenrate von 40 Gbit/s, vorzugsweise bis zu 270 Gbit/s. Im Vergleich zu den bekannten Ansätzen, beispielsweise den kapazitiven Verfahren (die auf ca. 10 GBit pro Sekunde begrenzt sind), können deutlich höhere Datenraten erreicht werden.

Hierbei werden über einen einzelnen Kanal beispielsweise 65 GBit/s übertragen, basierend auf einer spektralen Datendichte von 3 Bit pro Sekunde und Hertz und einer Standard-V-Band-Bandbreite von 25 GHz (einschließlich Schutzintervallen). Das V-Band ist ein Frequenzbereich im Mikrowellenspektrum, der von 50 GHz bis 75 GHz reicht. Es wird von der IEEE (Institute of Electrical and Electronics Engineers) als Standardbezeichnung verwendet.

Um eine Datenrate von mehr als 40 GBit/s zu erreichen, ist je nach gewähltem Modulationsschema eine Bandbreite von mehr als 15 GHz erforderlich.

Folglich muss das System ein relativ flaches Amplitudenverhältnis aufweisen.

Gemäß den Frequenzregulierungsgesetzen der ITU (International Telecommunication Union) und den besonderen Ausbreitungseigenschaften von Radiowellen in der Atmosphäre können nur Frequenzen oberhalb von 51,4 GHz ohne Sondergenehmigung verwendet werden, um eine Bandbreite von 15 GHz oder mehr zu erreichen.

Daher sind das V-Band und das E-Band, das von 60 bis 90 GHz reicht, eine praktikable Option.

Die Erfindung beruht auf Grundsätzen, die auf Strukturen für beliebige Bänder anwendbar sind, wobei die Skalierbarkeit passiver Komponenten berücksichtigt wird, aber durch mechanische Fertigungstoleranzen eingeschränkt ist. Der Klarheit halber wird das erfindungsgemäße Konzept in der folgenden Diskussion für das V-Band betrachtet.

Das System muss eine relativ geringe spezifische Einfügedämpfung pro Längeneinheit über die angegebene Bandbreite aufweisen. Ein Verlustwert sprich eine Einfügedämpfung von mehr als 40 dB/m wird als hoch angesehen.

Eine Erhöhung des Reichweitenbereichs durch die Anwendung von Automatic Gain Controll kurz AGC (Automatische Verstärkungssteuerung) für das Sender-zu-Empfänger-System kann dann erforderlich werden.

Da die Erfindung auf einem Hohlleiter 101 basiert, ist es entscheidend, nur eine einzelne Modenwelle innerhalb des Hohlleiters 101 zu nutzen und damit die Modendispersion so weit wie möglich zu minimieren. Dadurch wird sichergestellt, dass die Impulsantwort des Kanals so kurz wie möglich bleibt. Würde man eine Mehrmodenwelle verwenden, würde dies aufgrund der unterschiedlichen Ausbreitungsgeschwindigkeiten (d.h. der Dispersion) der verschiedenen Moden und Echos zu einer langen Impulsantwort mit mehreren Echos führen.

Bei CT-Anwendungen muss das Datenübertragungssystem um den Umfang des Drehübertragers mit einem Durchmesser von mehr als 1,2 m angeordnet sein. Bei der Konfiguration des CT drehen sich Transmitter 7 und Receiver 8 mit einer vorbestimmten Geschwindigkeit gegeneinander, beispielsweise mit bis zu 300 Umdrehungen pro Minute (U/min). Unter Berücksichtigung eines Ringdurchmessers von mindestens 1,2 Metern und eines Betriebs der RF-Kommunikation im V-Band verursacht diese Drehbewegung durch die relative Bewegung einen erheblichen Dopplereffekt, der zu Frequenzverschiebungen von bis zu 5 kHz führen kann.

Der typische Zwischenraum zwischen Rotor 102 und Stator 103 beträgt etwa 1 mm, was ihre relative Drehung ermöglicht. Dieser Zwischenraum wird jedoch durch die axiale Verschiebungsgenauigkeit des Drehübertragers und die Lagertoleranzen begrenzt. Die Verringerung dieses Zwischenraums ist entscheidend für die Minimierung elektromagnetischer Signalverluste. Obwohl die Verwendung eines speziellen Lagers für das Datenübertragungssystem unerlässlich ist, kann damit nur eine Verringerung des Zwischenraums auf etwa 0,6 mm bis 0,2 mm erreicht werden.

Wenn man standardmäßige einzelne viereckige Hohlleiter 101 in einem Drehübertrager verwendet, kommt es aufgrund des Zwischenraums in der Struktur zu unerwünschten Leckagen zwischen den Oberflächen von Rotor 102 und Stator 103. Diese Leckage ist direkt proportional zur Größe des Spalts 6, 106 relativ zu den Wandabmessungen des Hohlleiters 101 und ist daher unerwünscht, da sie die Einfügungsdämpfung erhöht.

Erfindungsgemäß wird daher ein Steg-Wellenleiter-Konzept verwendet, weil es das elektromagnetische Feld hauptsächlich zwischen den Stegen 105 bündelt und so größtenteils verhindert, dass das Feld durch den Spalt 6, 106 austritt.

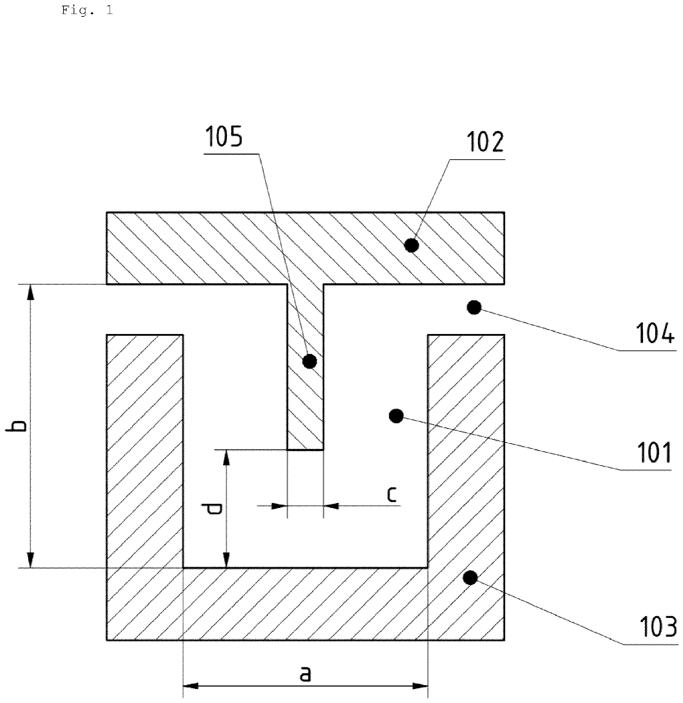

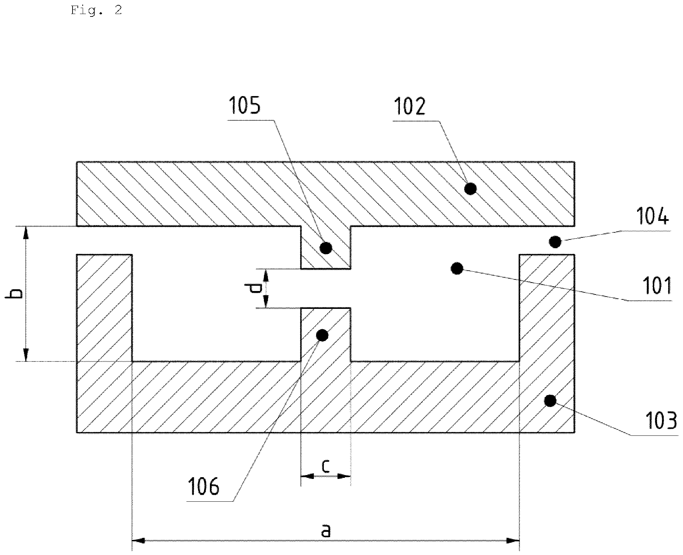

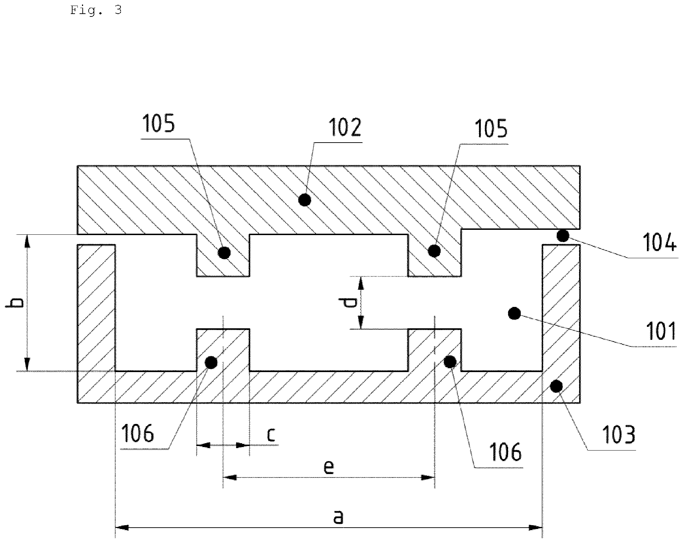

Es werden drei grundlegende Varianten des Steg-Welleleiters vorgeschlagen, nämlich eine Konfiguration mit einzelnen Stegen (SRWG) - siehe Fig. 1 und Fig. 2, mit zwei Stegen (DRWG) - siehe Fig. 3 - und mit vier Stegen (QRWG), wobei in diesen Ausführungsformen viereckige Stege 105 und rechteckige Hohlleiter 101 verwendet werden.

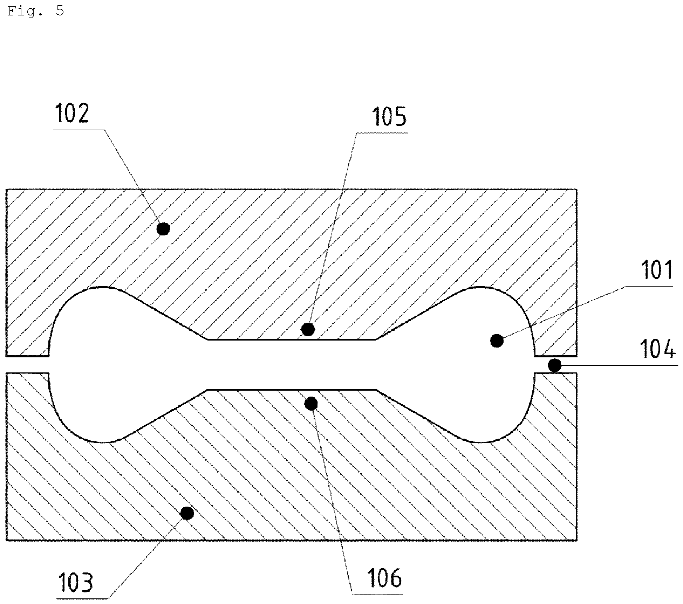

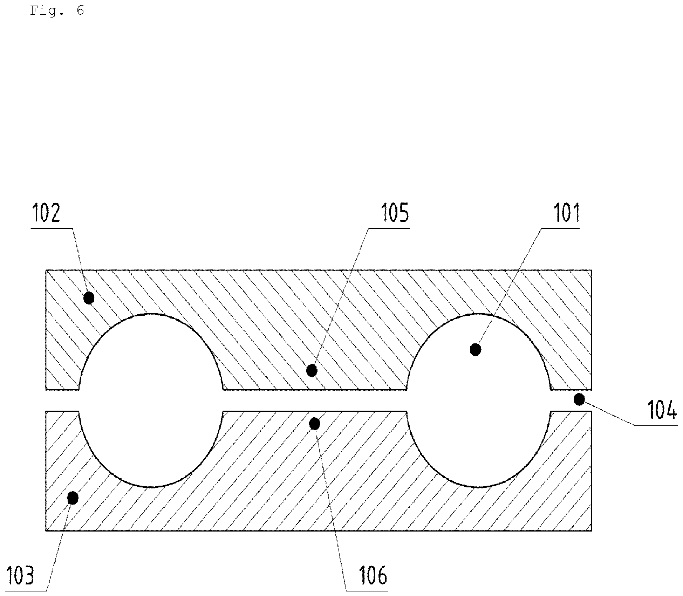

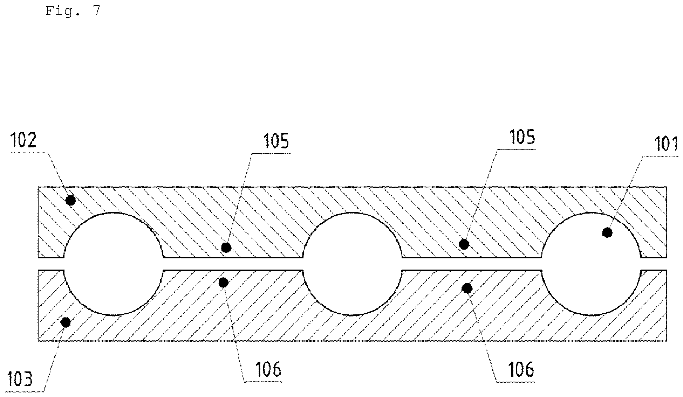

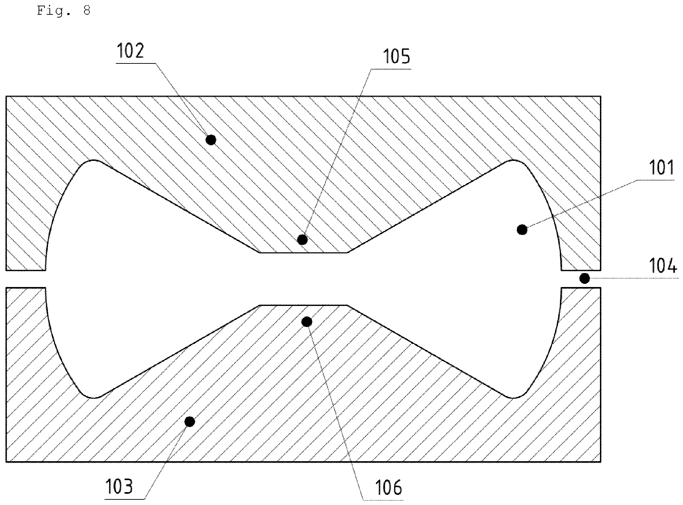

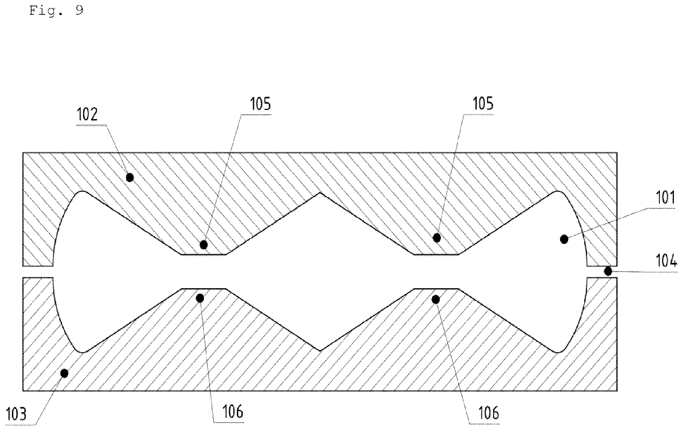

Wie in den Figuren 5, 6, 7, 8 und 9 gezeigt ist, ist die Erfindung hierauf nicht beschränkt, und es können andere Formen sowohl für die Stege 105 als auch für die Hohlleiter 101 verwendet werden.

Der Hohlleiter 101 soll mit einer Terminierung 112 aus absorbierendem Material abgeschlossen werden. Dies ist wichtig, um die Ausbreitung von Mehrfachechos durch den um die gemeinsame Drehachse ringförmig ausgeführten Hohlleiter 101 zu verhindern.

Die Grundstruktur des Hohlleiters 101 wurde durch Anpassung der Hauptabmessungen optimiert, wie es in Tabelle 1 angegeben ist.

Erfindungsgemäß wird das elektromagnetische Feld zwischen den Stegen 105 gebündelt, so dass fast kein Feld durch den Spalt 6, 106 austritt. Dabei zeigt sich, dass eine Leckage bei der Konfiguration mit nur einem Steg 105 in den Figuren 1 und 2 höher ist als bei der Ausführungsform mit zwei Stegen 105 in der Figur 3.

Tabelle 1 zeigt berechnete Abmessungen in mm der erfindungsgemäßen Konfigurationen für das V-Band.

| | Einzelner Steg | Doppelter Steg | Vierfacher Steg |
|---|---|---|---|
| längste Wand | 3.39 | 5.01 | 8.08 |
| kürzeste Wand | 2.24 | 2.2 | 2.19 |
| Breite des Stegs | 0.2 | 1 | 0.83 |
| Zwischenraum zwischen den Stegen | 0.65 | 0.75 | 0.7 |
| Trennung der Ränder | - | - | 2.84 |

Bei Hohlleiter 101 mit einem einzelnen Steg 105 ist "d" der Abstand zwischen dem Steg 105 und der gegenüberliegenden Wand bzw. innerhalb eines zweiteiligen Stegs 105 in Fig. 2.

Die Einspeisung in den Hohlleiter 101 kann durch eine Kombination aus einer T-Verzweigung und pyramidalem Hornübergang erreicht werden, dessen Breite auf die Breite des Hohlleiters abgestimmt ist. Dies gewährleistet die Einspeisung im Einzelmode und die Ausbreitung durch Umwandlung der Hauptmode (TE10) in den gewünschten Mode des Hohlleiters 101.

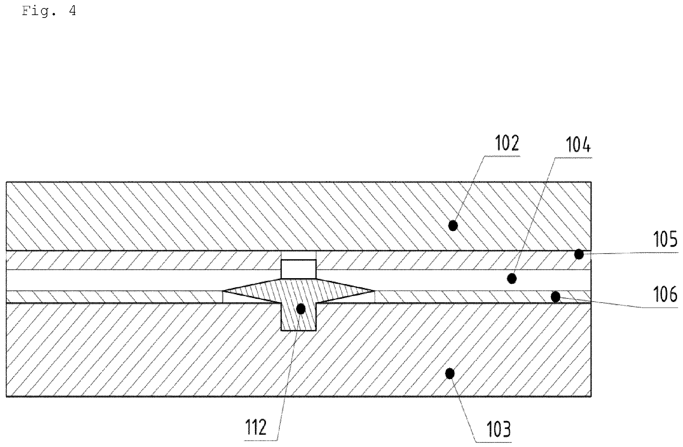

Bei einer bevorzugten Ausführungsform, die in Fig. 4 gezeigt ist, ist eine Terminierung 112 so konzipiert, dass sie elektromagnetische Wellen absorbiert und Reflektionen minimiert. Die Terminierung 112 hat die Form zweier an der Basis verbundenen Pyramiden und ist aus für elektromagnetische Wellen absorbierendem Material gefertigt.

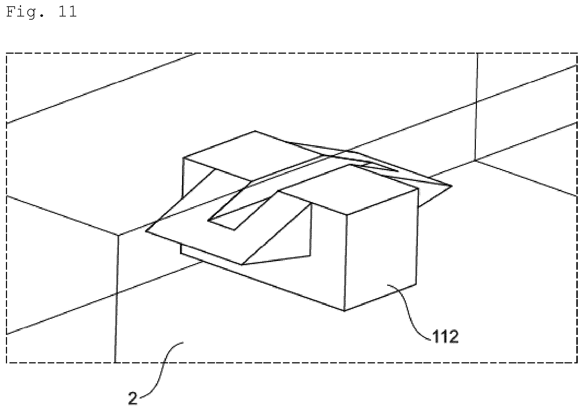

Die grundsätzliche Geometrie der Terminierung 112 oder des Absorbers ist in Figur 11 dargestellt. Sie umfasst eine absorbierende Wand 210, die eine effiziente Absorption gewährleistet, und einen passenden pyramidenförmigen Stumpf 211, der die Reflexionen verringert. Das gleiche Prinzip liegt anderen Geometrien zugrunde, die ähnliche Leistungsmerkmale ausführen.

Beispielsweise kann die Höhe der Pyramide 5 mm und die Wanddicke des Absorbers 4 mm betragen.

Andere Hohlleiterprofile sind möglich, wie dies in den Figuren gezeigt ist. Es ist möglich, dass Formen wie Hundeknochen-, Schmetterlings- oder Hanteldesigns die HF-Eigenschaften, einschließlich Einfügungs- oder Rückflussdämpfung, verbessern.

Vorangehend wurde die Erfindung für eine Übertragung im V-Band beschrieben. Sie ist hierauf aber nicht beschränkt. Auch Übertragungen im E-Band oder im Ka-Band, im Frequenzbereich von 27 bis 40 GHz, können mit entsprechender Isolierung und Abschirmung verwendet werden.

Die einzelnen Komponenten zur Signalverarbeitung sind im Ka-Band kostengünstiger als im V-Band, allerdings muss das System so isoliert werden, dass keine Störungen nach außen auftreten können. Daher bietet das V-Band den Vorteil, dass es keine aufwendige Isolation erfordert.

### Bezugszeichenliste

1 Gantry
2 Hohlleiterübertragungssystem
3 Röntgenröhren
4 Röntgendetektoren
5 Patienten
6 Spalt
7 Transmitter
8 Receiver
9 Computer
101 Wellenleiter
102 Rotor
103 Stator
104 Spalt
105 Steg
112 Terminierung
a Breite Stator innen
b Abstand Rotor zu Stator
c Breite Steg
d Abstand Stator zu Steg
e Abstand Stege

## Patentansprüche

1. Datenübertragungssystem zur Übertragung von Daten zwischen zwei relativ zueinander um eine gemeinsame Achse drehbar gelagerten Teilen (102, 103), wobei eines der Teile (102) wenigstens einen Transmitter (7) und das andere Teil (103) einen Receiver (8) umfasst, wobei das Datenübertragungssystem einen Hohlleiter (101) aufweist, der in einer Längsrichtung geteilt ist und im Querschnitt mindestens einen Steg (105) aufweist.

2. Das Datenübertragungssystem nach Anspruch 1, wobei der Hohlleiter (101) mit einem einzelnen Steg (105) in der Mitte des Hohlleiters (101) entlang einer breiten Wellenleiterwand ausgebildet ist.

3. Das Datenübertragungssystem nach Anspruch 1, wobei der Hohlleiter (101) mit mehreren Stegen (105) ausgebildet ist.

4. Das Datenübertragungssystem nach einem der Ansprüche 1 bis 3, wobei der Hohlleiter (101) auf einer Seite des Transmitters (7) mit einer Terminierung (112) aus einem absorbierenden Material ausgestattet ist.

5. Das Datenübertragungssystem nach einem der vorherigen Ansprüche, wobei die mindestens eine Terminierung (112) pyramidenförmig ist.

6. Das Datenübertragungssystem nach einem der vorherigen Ansprüche, wobei die Querschnittsform des Hohlleiters (101) und/oder das Anregungsprinzip des Hohlleiters (101) für eine Einzel-Moden-Welle konfiguriert sind.

7. Das Datenübertragungssystem nach einem der vorherigen Ansprüche, wobei der Hohlleiter (101) einen Querschnitt hat, der ausgewählt ist aus der Gruppe aus runden, ovalen, schmetterlingsförmigen, T-förmigen, doppel-T-förmigen oder eckigen Querschnitten.

8. Das Datenübertragungssystem nach einem der vorherigen Ansprüche, wobei mehrere parallel angeordnete ringförmig verlaufende Hohlleiter (101) vorgesehen sind.

9. Das Datenübertragungssystem nach einem der vorherigen Ansprüche, wobei mindestens einer der ringförmig verlaufenden Hohlleiter (101) in mehrere Kreissegmente unterteilt ist, auf denen Signale verschiedener Transmitter übertragen werden können.

10. Das Datenübertragungssystem nach einem der vorherigen Ansprüche, wobei mehrere Teile des Hohlleiters (101) als Phased-Array ausgeführt sind.

11. Computertomograph umfassend das Datenübertragungssystem nach einem der vorherigen Ansprüche.
